# FASCICULE DE BREVET EUROPEEN

(11) **EP 1 412 753 B1**
(45) Date de publication et mention de la délivrance du brevet: **12.09.2007**
(21) Numéro de dépôt: 02764947.4
(22) Date de dépôt: 05.07.2002
(51) Int. Cl.: G01N 33/543, G01N 21/64

(54) **BIOPUCE ET SON PROCEDE DE FABRICATION**
BIOCHIP UND VERFAHREN ZU SEINER HERSTELLUNG
BIOCHIP AND THE PRODUCTION METHOD THEREOF

(30) Priorité: 11.07.2001 FR 0109245
(43) Date de publication de la demande: 28.04.2004
(73) Titulaire: CENTRE NATIONAL DE LA RECHERCHE SCIENTIFIQUE (CNRS), 75794 Paris Cedex 16 (FR); ECOLE CENTRALE DE LYON, 69131 Ecully Cédex (FR)
(72) Inventeur: MARTIN, Jean-René, F-69380 Lozanne (FR); GARRIGUES, Michel, F-69890 La Tour de Salvagny (FR); CHAUVET, Jean-Paul, F-69370 Saint Didier au Mont d'OR (FR); BRAS, Marlène, 38000 Grenoble (FR); BESSUEILLE, François, F-69830 Saint Georges (FR); SOUTEYRAND, Eliane, F-41120 Cellettes (FR); CABRERA, Michel, F-69008 Lyon (FR); CLOAREC, Jean-Pierre, F-69001 Lyon (FR)
(74) Mandataire: Jacquard, Philippe Jean-Luc
(86) Numéro de dépôt international: PCT/FR2002/002365
(87) Numéro de publication internationale: WO 2003/008975

(56) Documents cités:
- WO-A-96/26432
- WO-A-99/45354
- US-A- 4 882 288
- US-A- 5 812 272
- US-A- 5 866 433
- US-A- 6 008 892

## Description

La présente invention a pour objet une biopuce d'un type permettant une détection de sondes biologiques ou biochimiques par fluorescence, ainsi que son procédé de fabrication.

Les biopuces sont des outils d'analyse très performants dans les domaines touchant à la biologie moléculaire. Leur technologie d'élaboration est progressivement, en train de se mettre en place. Leur utilisation permettra d'accélérer la Recherche et débouchera, en particulier, sur de nouvelles techniques de diagnostic.

Une biopuce est un ensemble de systèmes de substances biologiques de reconnaissance, telles que les molécules biologiques, fonctionnant en parallèle. Parmi les biopuces, les puces à ADN sont celles qui actuellement sont les plus développées et sur lesquelles sont fondées de grands espoirs pour décupler les efforts de reconnaissance dans le domaine de la Génétique Moléculaire.

Physiquement, les biopuces sont des structures constituées d'un support solide sur lequel sont distribuées des zones distinctes, en général de dimensions microscopiques, supportant des substances biologiques sondes identiques de reconnaissance, chaque zone ou site comprenant un seul type de substances, pouvant être d'origine biologique ou biochimique. La reconnaissance est assurée par une interaction affine spécifique entre la substance sonde fixée sur le support et la sustance cible contenue dans la solution à analyser. La lecture de la biopuce est réalisée par le marquage de la substance cible , au moyen d'un marqueur, le plus souvent, fluorescent. Lors de la reconnaissance, la substance cible apporte sur la zone supportant la substance sonde, son marqueur. La connaissance de la nature de la sonde permet de connaître la substance cible avec laquelle l'interaction affine s'est réalisée.

L'invention vise à améliorer, d'une part, la qualité technique de la biopuce et d'autre part à améliorer la qualité de la lecture par fluorescence.

La fixation de molécules, telles que des oligonucléotides ou des brins d'ADN, sur le support, pour la réalisation de la biopuce, fait appel à des réactions chimiques ayant lieu à la surface du solide. Le plus souvent, la molécule biologique sonde à fixer est en solution. Selon les propriétés de surface relatives du support et de la solution, qui contient les sondes à fixer, la solution déposée peut avoir la propension à s'étaler sur le substrat en rendant impossible l'obtention de zones d'intérêt de taille contrôlée et de faible dimension.

De la même façon, la synthèse directe localisée de molécules biologiques, telles que les oligonucléotides, peut être réalisée par le dépôt localisé, sur la surface du substrat, d'une partie des réactifs et notamment des phosphoramidites. Ces divers réactifs sont le plus souvent en solution dans un solvant tel que l'acétonitrile. Ce solvant a la propriété d'être très mouillant, ce qui entraîne, si l'on ne prend pas les précautions nécessaires, un étalement des réactifs sur la surface. Ainsi, ces propriétés empêchent d'obtenir la maîtrise de l'aire de synthèse et donc la définition géométrique des zones d'intérêt.

D'autre part, l'analyse est réalisée en mettant en contact la biopuce avec une solution qui contient les molécules cibles marquées, préalablement ou postérieurement au contact, avec un groupement fluorescent (ou éventuellement marquées de manière radioactive). La reconnaissance résulte d'un couplage biochimique relativement fort entre la sonde et l'analyte (par la formation de liaisons hydrogène, par exemple). L'analyte est ainsi fixé sur le plot de reconnaissance et peut être identifié par la connaissance de la sonde couplée, immobilisée sur ce plot ou site.

Parallèlement à cette reconnaissance spécifique, les molécules cibles peuvent, selon la nature du support, se fixer de manière non sélective sur celui-ci (le plus souvent par un processus d'adsorption). Ce phénomène parasite a pour effet de diminuer le rapport signal sur bruit de la lecture.

La présente invention a pour objet d'apporter une solution à ces problèmes pour améliorer la qualité biologique de la puce en jouant sur sa qualité optique.

De plus, selon une variante avantageuse, l'amélioration de la qualité physicochimique de la puce par une double fonctionnalisation localisée, peut permettre une meilleure définition géométrique des sites supportant les molécules biologiques sondes, tout en assurant l'identité de la taille des sites et leur alignement et en diminuant notablement l'adsorption non sélective des biomolécules cibles.

L'invention concerne ainsi une biopuce comprenant un substrat présentant une surface principale réfléchissante telle que définie dans la revendication 1. On entend par épaisseur optique, le produit de l'épaisseur physique effective par l'indice de réfraction de la couche transparente.

λ' désigne une longueur d'onde comprise entre λ₀ et λ₁, λ₀ désignant une longueur d'onde d'excitation de fluorescence et λ₁ une longueur d'émission de fluorescence.

La biopuce peut être caractérisée en ce que la surface principale présente des sites rendus sensibles à une détection de fluorescence par une couche transparente d'épaisseur optique (2k+1)λ₁/4, avec k entier positif nul, ce qui permet de favoriser l'émission fluorescente.

Préférentiellement, la surface principale est, en dehors des sites sensibles, recouverte d'une couche transparente d'épaisseur optique m λ₀/2, ce qui permet d'empêcher l'excitation de la fluorescence.

Selon un mode de réalisation avantageux, mettant en oeuvre une silanisation localisée, la biopuce est caractérisée en ce qu'en dehors des sites sensibles, la surface du substrat est recouverte d'une première couche mince, notamment une monocouche de molécules d'une première substance A, comportant une chaîne hydrocarbonée de type (CH₂)p avec 1 < p < 30 portant à une extrémité un groupement tel qu'un silane ou silanol permettant la formation d'une liaison covalente et à l'autre extrémité une fonction chimique stable et inerte, par exemple CH₃ ou ses dérivés halogénés. Ladite première substance est hydrophobe.

Selon un mode de réalisation préféré, la biopuce est caractérisée en ce que les sites sensibles sont recouverts d'une deuxième couche mince, notamment une monocouche de molécules d'une deuxième substance B présentant à une extrémité un groupement permettant une fixation par liaison covalente sur le substrat, par exemple une fonction silane ou silanol, et à l'autre extrémité un groupement apte à fixer de façon covalente une molécule sonde, par exemple la fonction acide COOH ou alcool OH.

Ladite deuxième substance est hydrophile.

La biopuce peut être caractérisée en ce que la surface du substrat est recouverte d'une couche de ladite substance B, et en ce qu'en dehors des sites sensibles, cette couche est recouverte d'une couche d'arrêt C.

L'invention concerne également un procédé de fabrication d'une biopuce telle que définie ci-dessus, caractérisé en ce qu'il met en oeuvre :
a) le dépôt sur un substrat en silicium d'une couche transparente d'épaisseur optique m λ'/2;
b) la réalisation dans ladite couche transparente de puits ou de plots dans lesquels l'épaisseur optique de la couche transparente est égale à (2k+1)λ/4, pour former lesdits sites sensibles.

Suivant les valeurs de k et de m, on pourra réaliser les sites sensibles sous forme de puits ou de plots.

Le procédé peut être caractérisé en ce qu'il met en oeuvre, dans le cas ou les zones sensibles sont des puits :
c) une double silanisation localisée par dépôt sélectif de ladite première couche mince, ce dépôt étant éventuellement suivi d'un traitement thermique ;
d) l'immersion du substrat dans une solution contenant la deuxième substance B.

Dans le cas ou les zones sensibles sont des plots, le procédé est caractérisé en ce qu'il met en oeuvre :
c') une double silanisation localisée par dépôt sélectif de la substance B sur les plots, ce dépôt étant éventuellement d'un traitement thermique ;
d') de l'immersion du support dans une solution contenant la substance A.

Selon un autre mode de réalisation, la double silanisation localisée est réalisée comme suit (après b) :
e) une couche de substance B est déposée sur la totalité de la surface du substrat par exemple par immersion dans la solution contenant la substance B. La fonction destinée à fixer la substance sonde est ensuite déprotégée et activée.
f) Une monocouche de molécules d'une substance d'arrêt C est ensuite déposée localement sur la surface, de manière à laisser les zones sensibles nues. Les molécules de la substance C comportent à une extrémité, une fonction qui réagit sur la fonction activée de la substance B. L'autre extrémité de la molécule comporte un groupement très inerte chimiquement,tel que le groupement méthyl ou encore un groupement méthyl substitué par le fluor. Ainsi, seules les zones sensibles activées seront aptes à fixer les substances sondes, la surface à l'extérieur des zones sensibles étant définitivement rendue inerte vis à vis de l'accrochage des substances biologiques.

Entre les groupements fonctionnels de la molécule de la substance C, il peut exister un chaîne organique telle qu'une chaîne aliphatique de type (CH₂)q où 0< q < 30.

Il est important de noter que les couches de fonctionnalisation ont des épaisseurs de l'ordre de 1 à 10 nanomètres et possèdent donc une épaisseur optique négligeable vis à vis de la couche transparente et ne jouent ainsi aucun rôle direct, sur les propriétés optiques de la biopuce.

Eventuellement, c et/ou d, et/ou e, et/ou f, peut être suivi d'un traitement thermique facilitant la silanisation.

D'autres caractéristiques et avantages de l'invention apparaîtront mieux à la lecture de la description qui va suivre en liaison avec les dessins ci-annexés dans lesquels les figures 1a à 1e illustrent un premier mode de réalisation du procédé de fabrication. Un mode préféré est illustré par les figures 1a à 1d, et 2a à 2c. Une variante est illustrée par la figure 3.

Le Brevet des Etats-Unis US 6,008,892 décrit une structure mettant à profit les propriétés optiques des lames minces : lorsqu'une couche transparente dont l'épaisseur optique est égale à un quart de la longueur d'onde ou un multiple impair du quart de la longueur d'onde, repose sur un substrat réféchissant (ou partiellement réfléchissant), l'émission de lumière par une source fluorescente émettant à cette longueur d'onde est maximale. Selon ce procédé, la surface complète du substrat est traitée de manière homogène, pour augmenter l'émission de fluorescence. La fluorescence parasite due aux substances biologiques adsorbées en dehors des zones sensibles est donc exacerbée de la même manière, que celles des zones sensibles, ce qui n'apporte aucune amélioration dans le rapport signal/bruit entre les zones sensibles et les zones non sensibles.

La présente invention remédie à cet inconvénient, grâce à une structuration de l'épaisseur de la couche transparente du substrat, qui permet d'exacerber la fluorescence des zones sensibles, et au contraire d'inhiber la fluorescence en dehors des zones sensibles.

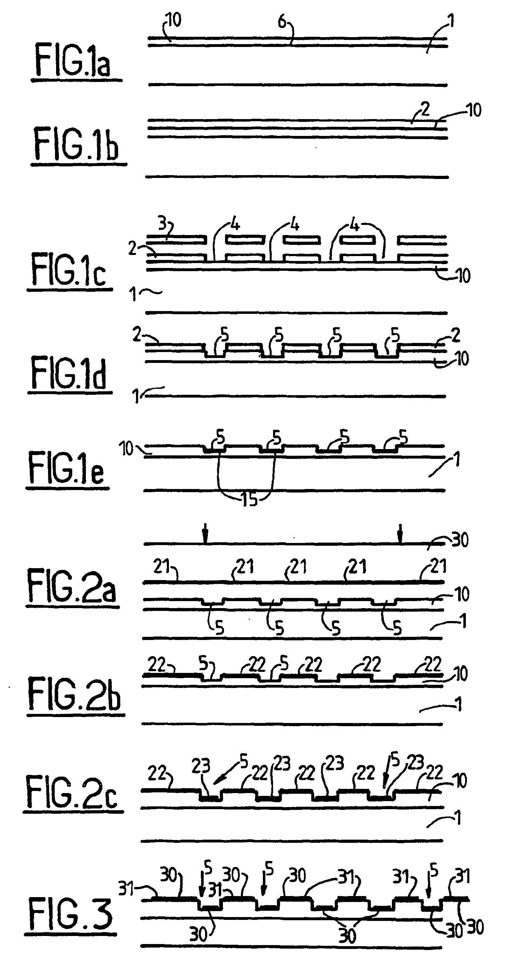

La structure optimisée selon l'invention est réalisée, à partir d'une couche 10 d'épaisseur optique λ/2 (ou plus généralement mλ/2) du matériau transparent déposée sur la surface 6 d'un substrat 1 réfléchissant à la longueur d'onde de lecture, par exemple une couche 10 en silice, ou en nitrure de silicium, sur un substrat en silicium, ou une couche d'oxyde métallique sur un substrat en métal, par exemple TiO₂ sur titane, ZrO₂ sur Zirconium, etc...(Figure 1a). Sur cette couche 10 est déposé un film 2 de résine photosensible (Figure 1b). La résine est insolée au travers d'un masque 3 qui permet d'ouvrir dans la résine 2, après révélation, des ouvertures 4 destinées à la formation des sites sensibles de la puce (Figure 1c). Une attaque chimique est alors conduite pour atteindre une épaisseur optique du film égale à (2k+1)λ/4 (Figure 1d) dans des zones localisées pour former des puits 5 constituant les sites sensibles de la puce.

Préférentiellement, on favorise l'émission de fluorescence par les sites sensibles en choisissant une couche transparente d'épaisseur optique (2k+1)λ₁/4. On favorise préférentiellement l'inhibition de l'excitation de la fluorescence en dehors des sites sensibles en choisissant une couche transparente d'épaisseur optique mλ₀/2.

Après élimination de la résine 2, un film d'un silane 15 comportant en extrémité, un groupement fonctionnel apte à immobiliser la molécule biologique peut être avantageusement déposé dans les puits 5 (Figure 1e). La structure est ensuite portée en température pour favoriser la réaction de silanisation. L'épaisseur de cette couche mince (généralement monomoléculaire) ne change pas de manière sensible les propriétés optiques de la structure.

Les molécules biologiques sondes sont ensuite fixées sur les différents sites sensibles par voie ex situ ou in situ. La surface supérieure libre de la couche subit enfin, un traitement qui a pour effet de neutraliser les groupements fonctionnels actifs qui n'ont pas réagi au cours de la phase précédente. Si toutefois, les molécules se fixent à l'extérieur des plots par débordement de la solution de dépôt, par exemple, le signal de fluorescence à l'extérieur du plot est de l'ordre de 200 fois plus faible que celui provenant de l'intérieur du plot et n'aura pas d'incidence, dans la plupart des cas sur la lecture.

Ainsi, la différence de chemin optique entre l'intérieur et l'extérieur des puits améliore le rapport signal sur bruit de la lecture de fluorescence en permettant de minimiser le signal de fluorescence hors des puits et en optimisant le signal de fluorescence dans les puits..

Il est possible d'obtenir une amélioration de la biopuce par l'amélioration conjointe de la qualité optique et de la qualité physicochimique du substrat.

Le substrat est micro-usiné par attaque chimique afin de réaliser les sites sensibles dans la couche comme décrit précédemment (Fig. 1a à 1d). Une opération de transfert est ensuite réalisée.

Après attaque chimique puis élimination de la résine (Fig. 1d), la puce est mise en contact avec une surface de transfert 30 sur laquelle est disposée une fine couche 21 d'un solvant contenant une substance de type A, par exemple, un silane hydrophobe (Fig. 2a). Le silane hydrophobe est ainsi transféré sur la surface supérieure de la puce pour former une mince couche 22 (Fig. 2b). Le dépôt de silane peut être encore réalisé par un dispositif d'encrage avec la solution contenant le silane. Un traitement thermique favorise la réaction de silanisation de la surface de la puce. Une deuxième étape de silanisation conduite, par exemple en plongeant la puce dans une solution contenant le deuxième silane, permet la silanisation par une mince couche 23 du fond des puits initialement nu (le deuxième silane ne se fixe pas sur le premier) (Fig. 2c). Le deuxième silane spécifique comporte à son extrémité libre un groupement fonctionnel connu en soi qui permet soit une liaison covalente avec la substance biologique afin de réaliser l'immobilisation de ladite substance biologique, soit le départ de la synthèse in-situ de la molécule biologique sonde : cette réaction de départ correspond également à l'établissement d'une liaison covalente.

L'épaisseur des couches minces 22 et 23 (tout au plus quelques couches moléculaires) est insuffisante pour modifier sensiblement les propriétés optiques de la surface qui cumule ainsi les propriétés de sélectivité procurées par les deux techniques.

Le procédé met ainsi en oeuvre un confinement physicochimique. Il consiste à délimiter les zones destinées à recevoir les sondes de caractère hydrophile, en déposant localement les molécules destinées à former une monocouche hydrophobe dans la région entre les sites sensibles, par micro-transfert. L'intérieur des sites sensibles est ensuite silanisé en utilisant un silane apte à permettre l'immobilisation des sondes préalablement synthétisées ou la synthèse in-situ des molécules biochimiques. Le micro-transfert de la mono-couche hydrophobe est réalisé grâce à l'utilisation d'une surface de transfert 30 qui peut être plane, ou bien être constitué par un micro-tampon présentant des régions de transfert en relief.

La surface de transfert 30 est mise en contact avec une solution contenant le silane hydrophobe. Une fine pellicule 22 de solution est alors transférée par contact en des endroits localisés de la couche 10 du substrat 1. Le substrat 1 est ensuite porté à une température de l'ordre de 100°C, afin de faciliter la réaction de silanisation. Grâce à un protocole spécifique et au choix d'une molécule appropriée, il est possible d'obtenir une monocouche organisée et compacte (SAM pour Self Assembled Monolayer). Les zones n'ayant pas reçu de silane sont alors traitées de manière à être recouvertes d'un autre silane qui possède à son extrémité libre un groupement fonctionnel, permettant la synthèse in situ d'oligonucléotides (groupement hydroxyle, par exemple) ou permettant l'immobilisation de molécules biologiques pré-synthétisés ou d'origine biologique, à l'aide d'un groupement fonctionnnel approprié (acide, par exemple). La réaction de greffage du second silane a lieu sur les groupements silanol de la silice ou du verre constituant le substrat mais elle ne peut pas avoir lieu sur la monocouche du premier silane fixé. Ainsi, seules les zones destinées à servir de sites sensibles seront fonctionnalisées.

De plus, le silane hydrophobe limite très fortement l'adsorption non sélective et de ce fait, améliore très sensiblement, le rapport signal sur bruit de la lecture par fluorescence (ou éventuellement par marquage radioactif), c'est-à-dire, en augmentant le rapport du signal vrai sur les zones supportant les sondes biologiques au signal éventuel provenant des zones nues sur lesquelles pourraient se produire une adsorption non sélective.

Il est en général possible de réaliser la même fonction avec des substances autres que le silane.

Il est en effet possible de déposer sur la partie supérieure du support, en dehors des sites sensibles une couche physiquement et chimiquement inerte qui limite l'adsorption et la fixation des biomolécules sondes lors de la fabrication des biopuces et l'adsorption ou la fixation des biomolécules cibles lors de l'analyse biologique, ce qui améliore le rapport signal/bruit de la mesure.

Cette couche inerte est constituée d'une molécule comportant une chaîne hydrocarbonnée de type (CH₂)p avec 1 <p< 30 ou un enchaînement polymérique, par exemple poly-éthylène-glycol ou autre ou un assemblage de différentes portion de chaînes, portant à une extrémité, un groupement permettant la formation d'une liaison covalente avec le support par exemple une liaison silane ou silanol, l'autre extrémité portant une fonction chimique stable et inerte telle que CH₃ ou ses dérivées halogénés substitués de tout ou partie des hydrogènes de la chaîne aliphatique (CH₂)p et de CH₃.. La couche formée peut être monomoléculaire ou polymérique selon la nature du premier groupement terminal. Le dépôt de la couche peut se faire avantageusement par contact avec une surface plane, préalablement recouverte d'une solution de cette molécule.

Il convient ensuite de déposer sur les sites sensibles une monocouche de molécules qui présentent une fonctionnalité différente à chacune de leurs extrémités : l'une permet sa fixation par liaison covalente sur le substrat, telle que la fonction silanol, et l'autre permet de fixer de façon covalente la biomolécule sonde par exemple la fonction acide COOH ou alcool OH. Cette monocouche permet alors d'immobiliser des biomolécules présynthétisées ou d'origine naturelle, ou encore de synthétiser, in situ dans les puits, les biomolécules sondes (oligonucléotides, PNA, protéine, etc...)

Un autre procédé est décrit en liaison avec les figures 3.

La surface du substrat est recouverte d'une couche 30 de la substance B, puis une couche 31 de la substance d'arrêt C est déposée localement en dehors des sites sensibles, ici les puits 5.

D'une manière préférée, l'efficacité de détection des sites sensibles est obtenue en utilisant deux techniques qui ont un effet complémentaire : l'effet physique associé à la différence de hauteur entre l'intérieur et l'extérieur des sites et l'effet physicochimique lié aux différences de propriétés de surface entre l'intérieur et l'extérieur des sites. De ce fait, la géométrie et la position relative des sites sensibles obtenues par une technique de type micro-technologie est parfaitement définie. Ceci contribue à la qualité de la puce et à l'amélioration de sa lecture.

Enfin, l'association des deux techniques, la structuration tridimensionnelle du support et la préparation de la surface pour permettre un accrochage préférentiel dans les sites sensibles, par exemple par double silanisation, permet d'améliorer la qualité de la puce et l'optimisation de sa lecture, en jouant sur quatre aspects qui sont mis en oeuvre séparément ou conjointement ::
- l'amélioration de la définition géométrique de la puce grâce à l'utilisation des techniques de masquage et ou de transfert,
- la diminution de la taille des sites sensibles grâce aux effets conjugués du confinement physicochimique et de l'usinage des sites.
- la diminution de l'adsorption non spécifique sur la zone entre les plots, grâce au dépôt par exemple d'une monocouche de silane hydrophobe par microtransfert,
- l'amélioration de la qualité de lecture optique de la puce grâce à l'optimisation des épaisseurs de la couche support transparente.

## Revendications

1. Biopuce comprenant un substrat présentant une surface principale réfléchissante **caractérisée en ce que** la surface principale (6) présente des sites localisés (5), **en ce que** ces sites localisés (5) sont rendus sensibles à une détection de fluorescence par une couche transparente d'épaisseur optique (2k+1)λ/4, avec k entier positif ou nul, λ désignant une longueur d'onde comprise entre une longueur d'onde λ₀ d'excitation de fluorescence, et une longueur d'onde λ₁ d'émission de fluorescence et **en ce qu'**en dehors des sites localisés sensibles (5), la surface principale (6) est recouverte d'une couche transparente d'épaisseur optique mλ'/2 avec m entier positif, λ', désignant une longueur d'onde comprise entre λ₀ et λ₁.

2. Biopuce selon la revendication 1, **caractérisée en ce que** ladite couche transparente qui recouvre les sites (5) rendus sensibles à une détection de fluorescence a une épaisseur optique égale à (2k+1)λ₁/4 avec k entier positif ou nul.

3. Biopuce selon une des revendications 1 ou 2, **caractérisée en ce qu'**en dehors des sites sensibles, la surface principale (6) est recouverte d'une couche transparente d'épaisseur optique mλ₀/2.

4. Biopuce selon une des revendications précédentes, **caractérisée en ce qu'**en dehors des sites sensibles (5), la surface (6) du substrat (1) est recouverte d'une couche mince (22), notamment une monocouche de molécules, d'une première substance (A) comportant une chaîne hydrocarbonée de type (CH₂)p avec 1 < p < 30 portant à une extrémité un groupement d'accrochage, tel qu'un silane ou silanol, permettant la formation d'une liaison covalente avec le substrat et à l'autre extrémité une fonction chimique stable et inerte, par exemple CH₃ et ses dérivés halogénés.

5. Biopuce selon la revendication 4, **caractérisée en ce que** ladite première substance (A) est un silane hydrophobe.

6. Biopuce selon une des revendications précédentes, **caractérisée en ce que** les sites sensibles (5) sont recouverts d'une couche mince (23), notamment une monocouche de molécules, d'une deuxième substance (B) présentant à une extrémité un groupement permettant une fixation sur le substrat par liaison covalente, par exemple une fonction silane ou silanol, et à l'autre extrémité un groupement apte à fixer de façon covalente une molécule sonde, par exemple un groupement présentant la fonction acide COOH ou alcool OH.

7. Biopuce selon la revendication 6, **caractérisée en ce que** ladite deuxième substance (B) est un silane hydrophile.

8. Biopuce selon une des revendications 1 à 3, **caractérisée en ce que** la surface du substrat est recouverte d'une couche de ladite deuxième substance (B) qui présente à une extrémité un groupement permettant une fixation sur le substrat par liaison covalente, par exemple une fonction silane ou silanol, et à l'autre extrémité un groupement apte à fixer de façon covalente une molécule sonde, et **en ce qu'**en dehors des sites sensibles (5) ladite couche de ladite deuxième substance (B) est recouverte d'une substance d'arrêt (C) qui présente à une extrémité une fonction qui réagit sur le groupement activé de la deuxième substance (B) et à une autre extrémité un groupement apte à la rendre chimiquement inerte vis-à-vis de l'accrochage des substances biologiques.

9. Procédé de fabrication d'une biopuce selon une des revendications précédentes, **caractérisé en ce qu'**il met en oeuvre:
a) le dépôt sur un substrat (1) d'une couche transparente (10) d'épaisseur optique mλ'/2;
b) la réalisation dans ladite couche transparente de puits ou des plots (5) dans lesquels l'épaisseur optique de la couche transparente est égale à (2k+1)λ/4, pour former lesdits sites sensibles.

10. Procédé selon la revendication 9, **caractérisé en ce qu'**il met en oeuvre pour des puits (5) :
c) le dépôt par transfert sélectif de ladite première couche mince (22) de ladite première substance (A);
d) l'immersion du substrat dans une solution contenant ladite deuxième substance (B).

11. Procédé selon la revendication 10, **caractérisé en ce que** c et/ou d est suivi d'un traitement thermique facilitant la formation d'une liaison covalente.

12. Procédé selon la revendication 9, **caractérisé en ce qu'**il présente après b) :
e) le dépôt sur toute la surface (6) du substrat (1) d'une couche mince (30) de ladite deuxième substance (B) et activation de la fonction destinée à fixer des molécules sondes
f) le dépôt d'une couche (31) de ladite substance d'arrêt (C) en dehors desdits sites sensibles (5).

## Claims

1. A biochip comprising a substrate presenting a reflecting main surface, the biochip being **characterized in that** the main surface (6) presents localized sites (5), **in that** these localized sites (5) are made sensitive to fluorescence detection by a transparent layer having optical thickness (2k+1)λ/4 where k is a positive or zero integer and where λ designates a wavelength lying in the range from a wavelength λ₀ at which fluorescence is excited and to a wavelength λ₁ at which fluorescence is emitted and **in that** outside the sensitive sites (5), the main surface (6) is covered in a transparent layer of optical thickness mλ'/2 where m is a positive integer, and λ' designates a wavelength lying in the range λ₀ to λ₁.

2. A biochip according to claim 1, **characterized in that** said transparent layer which covers the sites (5) that have been made sensitive to fluorescence detection has an optical thickness equal to (2k+1)λ₁/4 where k is a positive integer or zero.

3. A biochip according to claim 1 or claim 2, **characterized in that** outside the sensitive sites (5), the main surface (6) is covered in a transparent layer of optical thickness mλ'/2.

4. A biochip according to any preceding claim, **characterized in that** outside the sensitive sites (5), the surface (6) of the substrate (1) is covered in a thin layer (22), in particular a monolayer of molecules of a first substance (A) comprising a hydrocarbon chain of the (CH₂)p type with 1<p<30 carrying a catching-hold group such as a silane or a silanol at one end enabling a covalent bond to be made with the substrate, and carrying a chemical function at its other end that is stable and inert, e.g. CH₃ and halogenated derivatives thereof.

5. A biochip according to claim 4, **characterized in that** a first substance (A) is a hydrophobic silane.

6. A biochip according to any preceding claim, **characterized in that** the sensitive sites (5) are covered in a thin layer (23), in particular a monolayer of molecules of a second substance (B) presenting a group at one end suitable for fixing to the substrate by means of a covalent bond, e.g. a silane function or a silanol function, and presenting a group at its other end suitable for fixing in covalent manner to a probe molecule, e.g. a group presenting the acid COOH function or the alcohol OH function.

7. A biochip according to claim 6, **characterized in that** said second substance (B) is a hydrophilic silane.

8. A biochip according to any one of claims 1 to 3, **characterized in that** the surface of the substrate is covered in a layer of said second substance (B), which presents at one end a group suitable for fixing to the substrate by means of a covalent bond, e.g. a silane function or a silanol function, and presenting at its other end a group suitable for fixing in covalent manner to a probe molecule, and **in that** outside the sensitive sites (5), said layer of said second substance (B) is covered in a stop substance (C), which presents at one end a function that reacts with the activated group of the substance (B) and at its other end a group that is highly inert chemically against catching hold of biological substances.

9. A method of manufacturing a biochip according to any preceding claim, the method being **characterized in that** it implements the following steps:
a) depositing a transparent layer (10) of optical thickness mλ'/2 on a substrate (1); and
b) making wells or studs (5) in said transparent layer in which the optical thickness of the transparent layer is equal to (2k+1)λ/4, so as to form said sensitive sites.

10. A method according to claim 9, **characterized in that** it implements the following steps for the wells (5):
c) depositing said first thin layer (22) of the first substance A by selective transfer; and
d) immersing the substrate in a solution containing said second substance (B).

11. A method according to claim 10, **characterized in that** step c) and/or d) is followed by heat treatment to facilitate the formation of a covalent bond.

12. A method according to claim 9, **characterized in that** after step b), it presents the following steps:
e) depositing a thin layer (30) of said second substance (B) over the entire surface (6) of the substrate (1) and activating the function for fixing the probe molecules; and
f) depositing a layer (31) of a stop substance (C) outside said sensitive sites (5).

## Patentansprüche

1. Biochip umfassend ein Substrat, das eine reflektierende Hauptoberfläche aufweist, **dadurch gekennzeichnet, dass** die Hauptoberfläche (6) begrenzte Stellen (5) aufweist, **dadurch**, dass diese begrenzten Stellen (5) durch eine transparente Schicht mit einer optischen Dicke von (2k+1)λ/4 zur Fluoreszenzdetektion empfindlich gemacht sind, wobei k eine ganze positive Zahl oder null ist und λ eine Wellenlänge bedeutet, die zwischen einer Wellenlänge λ₀ liegt, bei der Fluoreszenz angeregt wird, und einer Wellenlänge λ₁, bei der Fluoreszenz emittiert wird, und **dadurch**, dass die Hauptoberfläche (6) außerhalb der empfindlichen begrenzten Stellen (5) mit einer transparenten Schicht mit einer optischen Dicke von mλ'/2 bedeckt ist, wobei m eine ganze positive Zahl ist und λ' eine Wellenlänge zwischen λ₀ und λ₁ bedeutet.

2. Biochip nach Anspruch 1, **dadurch gekennzeichnet, dass** die transparente Schicht, die die zur Fluoreszenzdetektion empfindlich gemachten Stellen (5) bedeckt, eine optische Dicke von (2k+1) λ₁/4 hat, wobei k eine ganze positive Zahl oder null ist.

3. Biochip nach einem der Ansprüche 1 oder 2, **dadurch gekennzeichnet, dass** die Hauptoberfläche (6) außerhalb der empfindlichen Stellen mit einer transparenten Schicht mit einer optischen Dicke von mλ₀/2 bedeckt ist.

4. Biochip nach einem der vorhergehenden Ansprüche, **dadurch gekennzeichnet, dass** die Oberfläche (6) des Substrats (1) außerhalb der empfindlichen Stellen (5) mit einer Dünnschicht (22), insbesondere einer Molekülmonoschicht, aus einer ersten Substanz (A) b edeckt ist, die eine Kohlenwasserstoffkette vom Typ (CH₂)p mit 1 < p < 30 aufweist, die an einem Ende eine Ankergruppe wie ein Silan oder ein Silanol trägt, die die Bildung einer kovalenten Bindung mit dem Substrat erlaubt, und am anderen Ende eine stabile und inerte chemische Funktion, beispielsweie CH₃ und seine halogenierten Derivate.

5. Biochip nach Anspruch 4, **dadurch gekennzeichnet, dass** die erste Substanz (A) ein hydrophobes Silan ist.

6. Biochip nach einem der vorhergehenden Ansprüche, **dadurch gekennzeichnet, dass** die empfindlichen Stellen (5) mit einer Dünnschicht (23), insbesondere einer Molekülmonoschicht, aus einer zweiten Substanz (B) bedeckt sind, die an einem Ende eine Gruppe aufweist, die eine Befestigung auf dem Substrat mittels kovalenter Bindung erlaubt, beispielsweise eine Silan- oder Silanolfunktion, und am anderen Ende eine Gruppe, die geeignet ist, ein Sondenmolekül kovalent zu binden, beispielsweise eine Gruppe, die eine Säurefunktion COOH oder eine Alkoholfunktion OH aufweist.

7. Biochip nach Anspruch 6, **dadurch gekennzeichnet, dass** die zweite Substanz (B) ein hydrophiles Silan ist.

8. Biochip nach einem der Ansprüche 1 bis 3, **dadurch gekennzeichnet, dass** die Oberfläche des Substrats mit einer Schicht aus der zweiten Substanz (B) bedeckt ist, die an einem Ende eine Gruppe aufweist, die eine Befestigung auf dem Substrat mittels kovalenter Bindung erlaubt, beispielsweise eine Silan- oder Silanolfunktion, und am anderen Ende eine Gruppe, die geeignet ist, ein Sondenmolekül kovalent zu binden, und **dadurch**, dass die Schicht aus der zweiten Substanz (B) außerhalb der empfindlichen Stellen (5) mit einer Blockierungssubstanz (C) bedeckt ist, die an einem Ende eine Funktion aufweist, die mit der aktivierten Gruppe der zweiten Substanz (B) reagiert, und am anderen Ende eine Gruppe, die sie für ein Anbinden von biologischen Substanzen chemisch inert macht.

9. Verfahren zur Herstellung eines Biochips nach einem der vorhergehenden Ansprüche, **gekennzeichnet durch**:
a) Abscheiden einer transparenten Schicht (10) mit einer optischen Dicke von mλ'/2 auf einem Substrat (1);
b) Erzeugen von Vertiefungen oder Kontakten (5) in der transparenten Schicht zur Bildung der empfindlichen Stellen, bei denen die optische Dicke der transparenten Schicht (2k+1)λ/4 beträgt.

10. Verfahren nach Anspruch 9, **gekennzeichnet durch** die folgenden Schritte für die Vertiefungen (5):
c) Abscheiden der ersten Dünnschicht (22) aus der ersten Substanz (A) **durch** selektiven Transfer;
d) Eintauchen des Substrats in eine Lösung, die die zweite Substanz (B) enthält.

11. Verfahren nach Anspruch 10, **dadurch gekennzeichnet, dass** nach c) und/oder d) eine Wärmebehandlung folgt, die die Bildung einer kovalenten Bindung erleichtert.

12. Verfahren nach Anspruch 9, **dadurch gekennzeichnet, dass** es nach Schritt b) umfasst:
e) Abscheiden auf der gesamten Oberfläche (6) von Substrat (1) einer Dünnschicht (30) aus der zweiten Substanz (B) und Aktivierung der zur Bindung der Sondenmoleküle vorgesehenen Funktion;
f) Abscheiden einer Schicht (31) aus der Blockierungssubstanz (C) außerhalb der empfindlichen Stellen (5).
